(19) Europäisches Patentamt
European Patent Office
Office européen des brevets

(11) Numéro de publication: **0 155 862**
**B1**

# (12) FASCICULE DE BREVET EUROPÉEN

(45) Date de publication du fascicule du brevet: **22.07.87**

(51) Int. Cl.⁴: **C 07 C 125/065,** C 07 D 295/20

(21) Numéro de dépôt: **85400252.4**

(22) Date de dépôt: **14.02.85**

## (54) Procédé de préparation de dérivés de l'acide carbamique.

(30) Priorité: **16.02.84 FR 8402327**
**16.02.84 FR 8402326**

(43) Date de publication de la demande:
**25.09.85 Bulletin 85/39**

(45) Mention de la délivrance du brevet:
**22.07.87 Bulletin 87/30**

(84) Etats contractants désignés:
**AT BE CH DE FR GB IT LI LU NL SE**

(56) Documents cité:
**US-A-3 763 217**

(73) Titulaire: **SOCIETE NATIONALE DES POUDRES ET EXPLOSIFS, 12, quai Henri IV, F-75181 Paris Cedex 04 (FR)**

(72) Inventeur: **Barcelo, Gérard, 3, rue Mozart Résidence Claire- joie, F-91700 Sainte- Geneviève-des- Bois (FR)**
Inventeur: **Senet, Jean- Pierre, 79, rue de la Gare Herbeauvilliers- Buthiers, F-77760 La Chapelle- la-Reine (FR)**
Inventeur: **Sennyey, Gérard, 1, Place des 4-Pavés Saint- Aubin, F-91190 Gif- sur- Yvette (FR)**

(74) Mandataire: **Jomard, Annick, Société Nationale des Poudres et Explosifs 12 quai Henri IV, F-75181 Paris Cedex 04 (FR)**

EP 0 155 862 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

L'invention concerne un nouveau procédé de préparation de dérivés de l'acide carbamique. Plus précisément elle concerne un procédé de préparation des dérivés de formule:

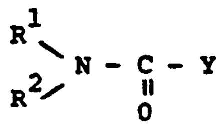

dans laquelle Y représente le reste d'un alcool, d'un mercaptan ou d'une amine, qui sont par conséquent des carbamates, thiocarbamates ou des urées.

Les procédés les plus couramment utilisés pour préparer ces composés sont par exemple pour les carbamates et thiocarbamates la réaction d'un chloroformiate ou thiochloroformiate sur une amine comme décrit dans l'article des Chemical Review de 1964, 64, pages 656-663 ou celle d'un chlorure de carbamyle ou d'un isocyanate sur un alcool, un phénol ou un mercaptan (Grignard - traité de Chimie Organiquetome XIV, page 20-31).

Les urées quant à elles sont obtenues le plus souvent par réaction d'un isocyanate ou d'un chlorure de carbamyle sur une amine. Lorsqu'elles sont symétriques elles peuvent également être préparées par phosgénation d'une amine (Grignard - tome XIV - pages 85,30).

Cependant, ces différents procédés ne permettent pas toujours de préparer les composés souhaités ou ils sont d'une mise en oeuvre difficile.

Certaines matières premières sont:

- instables, c'est le cas d'un certain nombre de chloroformiates tel que, par exemple, le chloroformiate de furfuryle, de tertiobutyle, de p-méthoxybenzyle;
- toxiques comme les isocyanates, le phosgène et surtout les chlorures de carbamyle légers qui sont cancérigènes,
- ou polluantes telles que les thiochloroformiates légers.

Des recherches pour trouver des voies nouvelles ont été effectuées.

Quelques composés ont été préparés par réaction du carbonate de diméthyle ou d'éthyle et de phényle sur l'aniline en présence d'un catalyseur comme le nitrate d'uranyle mais les rendements sont très faibles, il est nécessaire de chauffer le mélange à haute température: 100° (brevet US 3 763 217), les sous-produits sont obtenus en quantité importante.

Le carbonate de diphényle ne réagit pas sur les amines pour donner un carbamate, sauf en présence de catalyseur tel que l'hydroxy-2-pyridine (Noboru Yamazaki et Todao Igudi, Fukuji Higashi, Journal of Polymer Science - vol. 17 pages 835-841 (1979).

D'autres agents de carbamation ont été proposés tels que:

- les azides. Leur synthèse s'effectue en plusieurs étapes et est délicate. Ils peuvent se décomposer de façon explosive, comme l'azide de BOC (Angew, Chem., Ind. Ed. Engl. 16 1977 n°2),
- quelques essais ont été effectués avec des carbonates très particuliers tels que les carbonates mixtes de p-nitrophényle. Les produits secondaires obtenus sont difficiles à éliminer,
- les dicarbonates. Leur synthèse est très délicate et coûteuse. C'est en particulier le cas du dicarbonate de tertiobutyle. De plus, un reste protecteur est perdu,
- les fluoroformiates, mais leur préparation est difficile parce qu'elle impose le recours à des matières premières, non commerciales, délicates à manipuler, telles que ClCOF ou BrCOF.

La réaction de certains carbamates avec des amines a été étudiée. L'urée n'est obtenu qu'après chauffage à des températures élevées de l'ordre de 150° à 230° et à condition d'utiliser un catalyseur (Phillip Adams et Franck A. Baron, Chemical Review - 1965 page 574).

Dans ces différents procédés il se forme toujours un alcool ou un phénol qui est très souvent difficile à éliminer et la réaction est réversible.

Dans certains cas l'urée ne peut absolument pas être préparée. Ainsi par réaction du N-imidazole carbamate d'éthyle sur l'éthylamine, on obtient le N-éthyl carbamate d'éthyle et l'imidazole, et non l'urée.

Ce bref aperçu montre les limites des procédés classiques et le peu de résultats obtenus par les voies nouvelles.

Il était donc souhaitable de disposer d'un procédé général de préparation des dérivés de l'acide carbamique, plus facile à mettre en oeuvre tant en ce qui concerne l'emploi de composés de départ moins dangereux, que, les conditions opératoires et l'élimination des sous-produits.

Le procédé selon la présente invention est applicable à la préparation d'un grand nombre de dérivés de l'acide carbamique et convient particulièrement lorsque les autres voies d'accès à ces dérivés conviennent mal.

L'invention concerne un procédé de préparation des dérivés de l'acide carbamique de formule générale:

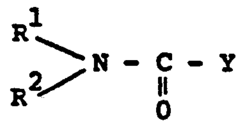

dans laquelle $R^1$, $R^2$ identiques ou différents représentent:
- un atome d'hydrogène,
- un radical aliphatique ou araliphatique, linéaire ou ramifié saturé ou insaturé, substitué ou non,
- un radical cycloaliphatique saturé ou insaturé substitué ou non,
- un radical hétérocyclique saturé ou non, substitué ou non, ou forment ensemble avec l'atome d'azote auquels ils sont liés un cycle substitué ou non, saturé ou non, qui peut comporter un ou plusieurs autres hétéroatomes et qui peut faire partie d'un système cyclique, Y représente les groupes OR, SR,

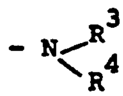

ou O-N =

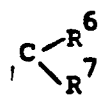

dans lesquels R représente un radical aliphatique ou araliphatique, linéaire ou ramifié, saturé ou insaturé, substitué ou non, un reste cycloaliphatique saturé ou insaturé, substitué ou non, un reste aromatique substitué ou non, $R^3$ et $R^4$ identiques ou différents représentent: un atome d'hydrogène, un radical aliphatique, araliphatique, cycloaliphatique ou heterocyclique, saturé ou non, substitué ou non, un radical aromatique substitué ou non, ou forment ensemble avec l'atome d'azote auquel ils sont liés, un hétérocycle, pouvant comporter un ou plusieurs autres hétéroatomes, substitué ou non, saturé ou non, et $R^6$ et $R^7$ identiques ou différents représentent un radical aliphatique ou cycloaliphatique, linéaire ou ramifié, substitué ou non, saturé ou non, ou représentent mais pas en même temps, un atome d'hydrogène, un radical alkylthio, un radical alkyloxy.

Ce procédé consiste à faire réagir en présence d'un accepteur d'acide halohydrique, à une température comprise entre -5° et 150°, un composé aminé hydrogéné de formule:

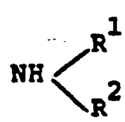

avec un dérivé α-halogéné de l'acide carbonique de formule:

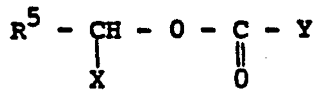

dans lesquelles $R^1$, $R^2$ et Y ont les significations precedentes, X représente un atome de fluor, chlore ou brome et $R^5$ représente un atome d'hydrogène, un reste aliphatique, araliphatique ou cycloaliphatique, saturé ou insaturé, substitué ou non, un reste aromatique substitué ou non.

On peut effectuer la réaction en présence ou non d'une solvant

Le schéma réactionnel peut s' écrire:

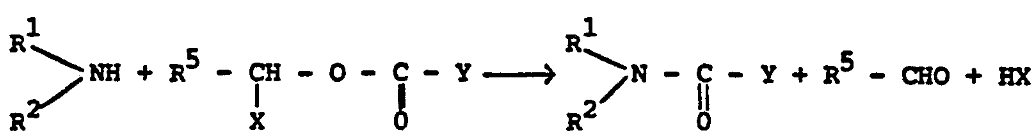

On constate que, de façon surprenante il y a élimination de HX, mais pas avec fixation du reste amine

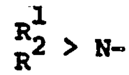

sur le carbone porteur de l'halogène pour former:

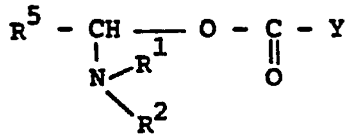

comme on pouvait normalement s'y attendre et comme c'est le cas, par exemple, dans la réaction d'un carbonate α-chloré avec un acide

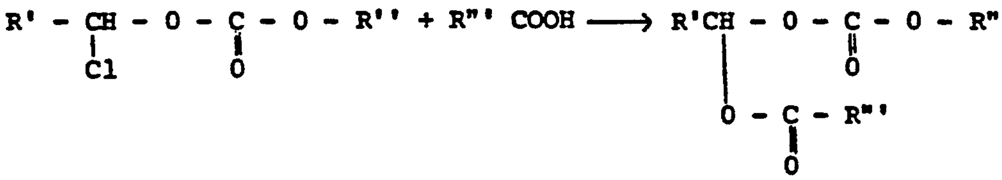

(ASTRA - Brevet FR 2 201 870)

Au contraire, selon notre procédé il y a coupure du dérivé α-halogéné, fixation du groupe

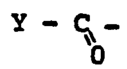

sur le reste du composé aminé et formation de l'aldéhyde $R_5$ CHO.

Comme composé aminé hydrogéné de départ, de formule:

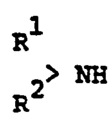

on peut utiliser l'ammoniac, la plupart des amines primaires ou secondaires connues.

De préférence lorsque $R^1$ ou $R^2$ représente un radical aliphatique, il comporte de 1 à 20 atomes de carbone. $R_1$ et $R_2$ peuvent également signifier un radical cycloaliphatique ou araliphatique, pouvant comporter jusqu'à 50 atomes de carbone, par exemple un benzyle, ou former ensemble un hétérocycle par exemple un cycle pipéridino, morpholino, imidazolyle.

Les substituants de $R^1$ et $R^2$ peuvent être des groupes variés tels que des groupes hydrocarbonés, des groupes fonctionnels acide, alcool, ester, éther, mercapto ou amino.

Comme amines intéressantes on peut citer la méthylamine, la diéthylamine, la di n-butylamine, l'isobutylamine, la n-octylamine, l'éthanolamine, la benzylamine, la N-méthyl N-benzylamine, la pipéridine, l'imidazole, l'hexaméthylèneimine, la morpholine, la diéthanolamine.

Les acides aminés naturels ou synthétiques optiquement actifs ou non ou racémiques utilisés en synthèse peptidique conviennent bien également.

On peut citer par exemple la L-phénylalanine, la L-proline, la glycine, la L-tyrosine, la L-sérine, l'acide L-aspartique, la proline, le glycinate d'éthyle, la phénylglycine.

Le deuxième composé de départ utilisé peut être un carbonate, thiocarbonate ou un carbamate α-halogéné. De préférence il est α-chloré.

Sa preparation peut être effectuee par differents procedes connus, par exemple, pour les dérivés chlorés par réaction d'un chloroformiate α-chloré de formule:

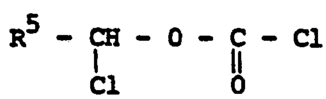

sur un compose hydroxylé ou sur un mercaptan, comme décrit dans l'article de M. Matzner, R. Kurkjy et R. J. Cotter (Chem. Rev. 64, pages 651-654 (1964)) ou sur une amine, par le procédé décrit dans la demande européenne n° 45 234 ou 83.401766.7.

Les chloroformiates α-chlorés eux-mêmes sont préparés très simplement par le procédé de phosgénation des aldéhydes revendiqué dans la demande européenne n° 40153.

Le radical $R^5$ est de préférence un radical léger tel qu'un radical aliphatique constitué de 1 à 4 atomes de carbone qui peut être substitué, de préférence par des atomes d'halogène et surtout de chlore. Les radicaux méthyle et trichlorométhyle sont particulièrement apprécies.

Le radical R du carbonate ou thiocarbonate est très varié. Ce peut être

- un radical aliphatique de préférence comportant de 1 à 12 atomes de carbone, tel qu'un méthyle, éthyle ou tertiobutyle, qui peut être substitué par exemple par un radical hétérocyclique tel qu'un furyle,
- un radical araliphatique par exemple un benzyle,
- un noyau aromatique substitué ou non faisant ou non partie d'un système cyclique, tel que le phényle, le 2,3 dihydro-2,2 diméthyl-7 benzofuranyle.

Le radical R dans toutes ses significations peut-être substitué par un ou plusieurs groupes

$$-O-\underset{\Vert \atop O}{C}-O-\underset{| \atop X}{CH}-R^5 \quad \text{ou} \quad -S-\underset{\Vert \atop O}{C}-O-\underset{| \atop X}{CH}-R^5$$

C'est en particulier lorsque l'amine de départ est un acide aminé, un des groupes protecteurs de la fonction amine habituellement utilisé dans la synthèse peptidique, tels que le tertiobutyle, le benzyle, le paranitrobenzyle, le 9-fluorénylméthyle, le trichloro-2,2,2 éthyle, le triméthylsilyléthyle, le furfuryle. Les carbonates α-chlorés et de tertiobutyle sont dans ce cas particulièrement appréciés.

$R^3$ et $R^4$ présents dans le carbamate α-chloré de départ représentent par exemple un atome d'hydrogène, le radical méthyle ou forment ensemble et avec l'atome d'azote auquel ils sont attachés un cycle imidazolyle. Ainsi l'α-chloroéthoxycarbonylimidazole et le N-méthylcarbamate de tétrachloro-1,2,2,2 éthyle sont particulièrement appréciés.

$R^6$ et $R^7$ représentent en particulier un atome d'hydrogène, un radical méthylthio, un radical aliphatique en $C_1$ à $C_{12}$ ou cycloaliphatique pouvant comporter 30 atomes de carbone. Les substituants de $R^6$ et $R^7$ peuvent être des radicaux hydrocarbonés, des groupes comprenant des hétéroatomes, en particulier le soufre.

Un deégagement d'hydracide halogéné HX se produisant au cours de la réaction pour la présence d'un réaction, pour réaction éliminer pour éliminer accepteur d'acide est nécessaire éliminer cet acide.

L'accepteur d'acide peut être une base organique ou minérale.

Parmi les bases préférées on peut citer l'hydroxyde de sodium ou de potassium, le carbonate ou bicarbonate de sodium ou de potassium, l'oxyde de magnésium, le sulfite de sodium qui sont généralement mis en oeuvre sous forme de solutions aqueuses, les amines tertiaires telles que la triéthylamine, la pyridine ou la N,N-diméthylaniline, l'amine de départ elle-même de formule

$$\begin{matrix} R^1 \\ R^2 \end{matrix} > NH.$$

La quantité de substance basique introduite dans le milieu doit être suffisante pour neutraliser la totalité de l'acide libéré. On utilise de préférence un léger excès par rapport à la quantité stoechiométrique.

La réaction selon l'invention est de préférence effectuée en milieu solvant. on utilise généralement un ou plusieurs solvants inertes vis-à-vis des réactifs. Om les choisit de préférence parmi les solvants aliphatiques chlorés tel que le dichlorométhane ou le dichloro-1,2 éthane, les éthers cycliques ou non, par exemple le tétrahydrofuranne ou le dioxanne, l'acétone, la pyridine, l'acétonitrile, le diméthylfornamide, les alcools tels que l'éthanol ou le tertiobutanol. Le milieu réactionnel peut contenir une certaine quantité d'eau, nécessaire par exemple à la dissolution des bases minérales.

La température de réaction dépend de la nature du solvant et de la réactivité des composés de départ. Elle est comprise entre -5° et 150°C. Elle est le plus souvent comprise entre 0° et 30°C pour la réaction des carbonates et thiocarbonates avec les amines et entre 30° et 100°C pour la réaction des carbamates.

Les composés de départ sont généralement utilisés en quantité stoechiométrique. Il est préférable d'utiliser un léger excès d'un des deux réactifs.

Lorsque l'amine de départ est utilisé comme accepteur d'acide, on utilise au moins deux équivalents d'amine par groupement

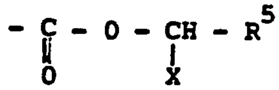

à transformer.

L'ordre d'introduction des réactifs n' e et pas une caractéristique fondamentale de l'invention. Cependant lorsque l'amine est primaire et qu'elle est également employée comme base acceptrice d'acide on préfère l'introduire à la suite de l'autre composé de départ.

Le procédé de l'invention permet l'obtention aisée de nombreux composés dont certains sont préparés avec beaucoup de difficulté par les méthodes habituelles. Ces composés sont très utiles comme produits pharmaceutiques, comme pesticides tels le CARBOFURAN, le N-méthylcarbamate de diméthyl-3,4 phényle, l'ALDICARBE, le CARBARYL pour les carbamates, le BUTYLATE, L' EPTC, le MOLINATE pour les thiocarbamates, le CHLORTOLURON, le MONURON pour les urées, ou comme intermédediaires de synthèse peptidique tels les carbamates d'acides aminés, par exemple, on peut citer la synthèse de l'ASPARTAM (Tetrahedron, Vol. 39 n° 24, pages 4121 à 4126 - 1983 - B. Yde & Al.)

L'invention est illustrée par les exemples qui suivent.

**Exemple 1** - Préparation du N,N-di-n-butylcarbamate d'éthyle

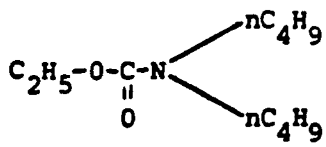

On ajoute, goutte à goutte, une solution de 26 g (0,2 mole) de di n-butylamine dans 20 ml de tétrahydrofuranne(THF) anhydre à une solution de 15,2 g (0,1 mole) de carbonate d'α-chloroéthyle et d'éthyle

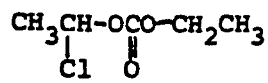

dans 80 ml de THF.

L'addition est réalisée sous agitation à 20°C. La réaction est, légèrement exothermique et on observe la formation d'un précipité de chlorhydrate de dibutylamine.

On agite pendant deux heures à 20°C, on élimine le précipité par filtration et on évapore le THF.

On reprend le résidu avec 200 ml de dichlorométhane, on lave avec 50 ml d'une solution aqueuse saturée de $KHCO_3$ puis avec 50 ml d'eau. Après séchage sur sulfate de magnésium, on élimine le solvat par évaporation et on distille sous pression réduite. On obtient ainsi 15,1 g (rendement 75 %) du carbamate attendu.

Point d'ébullition (Pt Eb): 78-80°C/40 Pa (0,3 mm Hg)

I R: $\nu C = 0$ : 1700 $cm^{-1}$

RMN $H^1$: 0,9-1,7 ppm (17 H) massif $C-CH_2-CH_3$
3,2 ppm (4 H) triplet $N-CH_2$
4,1 ppm (2 H) quadruplet $O-CH_2$

**Exemple 2** - Préparation du N-n-octylcarbamate d'éthyle

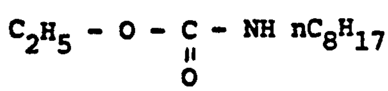

On ajoute une solution refroidie à 5-10°C de 7,6 g (0,05 mole) de carbonate d'α -chloroéthyle et d'éthyle dans 10 ml de THF à une solution refroidie également à 5-10°C de 12,9 g (0,1 mole) de n-octylamine dans 40 ml de THF.

Après l'addition effectuée sous agitation, on laisse revenir le mélange à la température ambiante et on le maintient à cette température pendant 2 heures sous agitation.

Après l'élimination du précipité par filtration et évaporation du solvant, on reprend avec 50 ml d'éther éthylique, on filtre à nouveau et on lave la phase éthérée avec 50 ml d'eau.

Après séchage sur sulfate de magnésium, on élimine le solvant par évaporation et on distille sous pression

réduite.

On obtient ainsi 8,64 g (rendement 86 %) du carbamate attendu.

Pt Eb: 110° C/40 Pa (0,3 mm Hg)

I R: $\gamma$C = 0 1700 $cm^{-1}$
$\gamma$N - H 3300 $cm^{-1}$

RMN $H^{-1}$: 0,1 - 1,7 ppm (18 H) massif ($CH_2$) $CH_3$
3,2 ppm (2 H) quadruplet N-$CH_2$
4,1 ppm (2 H) quadruplet O-$CH_2$
5,2 ppm (1 H) massif 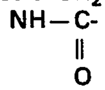

**Exemple 3** - Préparation du N-n-octylcarbamate d'éthyle

Dans un réacteur, on introduit 6,5 g (0,05 mole) de n-octylamine, 30 ml de THF, 10 ml d'eau et 10 g de carbonate de potassium $K_2CO_3$. On ajoute ensuite goutte à goutte sous agitation en maintenant la température à 5-10° C, 7,6 g (0,05 mole) de carbonate d'α-chloroethyle et d'éthyle en solution dans 5 ml de THF.

On laisse revenir le mélange à la température ambiante et on le maintient sous agitation à cette température pendant une heure. On ajoute 50 ml d'eau saturée en chlorure de sodium, on extrait avec 2 fois 40 ml d'éther éthylique, on rassemble et on sèche les phases étherées sur sulfate de magnésium.

Après élimination du solvant par évaporation et distillation sous pression réduite, on recueille 7,4 g (74 %) du carbamate attendu.

Pt Eb: 110-112° C/40 Pa (0,3 mm Hg).

**Exemple 4** - Préparation du N,N-di-n-butylcarbamate d'éthyle

Dans une solution de 6,5 g (0,05 mole) de di-n-butylamine et de 5,56 g (0,055 mole) de triéthylamine dans 40 ml de THF, on ajoute goutte à goutte, sous agitation, en maintenant la température à 5-10° C, 8,4 g (0,055 mole) de carbonate d'α-chloroéthyle et d'éthyle en solution dans 10 ml de THF.

On laisse revenir le mélange à la température ambiante et on le maintient sous agitation à cette température pendant 2 heures. Après élimination du précipité par filtration, évaporation du solvant et distillation sous pression réduite, on recueille 6,3 g (rendement 63 %) du carbamate attendu.

Pt Eb: 76° C/26,6 Pa (0,2 mm Hg)

**Exemple 5** - Préparation du N-n-octylcarbamate de tertiobutyle

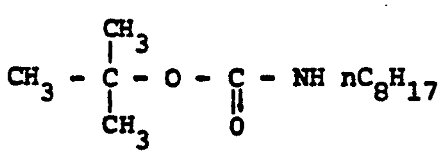

a/ Synthèse du carbonate d'a-chloroéthyle et de tertiobutyle

Dans un réacteur refroidi à + 5° C, on introduit 600 ml de dichloro méthane, 43,7 g (0,59 mole) de tertiobutanol et 94,7 g (0,66 mole) de chloroforniate d'α-chloroéthyle.

Om ajoute ensuite goutte à goutte, sous agitation, en maintenant la température entre 10 et 20° C, 57 g (0,72 mole) de pyridine.

On agite 4 heures à température ambiante.

Le milieu réactionnel est lavé avec 100 ml d'une solution aqueuse 1 N d'acide chlorhydrique, 200 ml d'une solution saturée en $Na_2CO_3$, 2 fois 100 ml d'eau glacée.

La phase organique est recueillie et séchée sur sulfate de magnésium. Après évaporation du solvant et distillation sous pression réduite, on obtient 91,5 g (86 %) de carbonate d'α-chloroéthyle et de tertiobutyle.

Pt Eb: 88° C/2,7 kpa (20 mm Hg)

I R: $\nu$C = 0 1750 $cm^{-1}$

RMN $H^{-1}$: 1,5 ppm (9 H) $(CH_3)_3$ - C - singulet
1,8 ppm (3 H) doublet $CH_3$ - C
|
Cl
6,9 ppm (1 H) quadruplet - O CH -
|
Cl

b/ Réaction du carbonate d'α-chloroéthyle et de tertiobutyle avec la n-octylamine

On ajoute goutte à goutte une solution de 52 g (0,4 mole) de n-octylamine dans 60 ml de THF anhydre à une solution de 36,2 g (0,2 mole) de carbonate d'α-chloroéthyle et de tertiobutyle dans 120 ml de THF.

L'addition est réalisée sous agitation à + 10°C.

On agite pendant environ quinze heures à température ambiante, on élimine les composés insolubles par filtration et on évapore le THF. On reprend le résidu avec 400 ml de dichlorométhane, on lave avec 100 ml d'une solution aqueuse 1 N d'HCl, 200 ml d'eau, 100 ml d'une solution aqueuse saturée de $KHCO_3$ puis avec 100 ml d'eau.

Après séchage sur sulfate de magnésium, on élimine le solvant par évaporation et distille sous pression réduite.

On obtient ainsi 36,52 g (rendement 80 %) du carbamate attendu.

Pt Eb: 142°C/200 Pa (1,5 mm Hg)

I R: $\nu$C = 0 1690 $cm^{-1}$
$\nu$NH 3340 $cm^{-1}$

RMN:$H^1$: 0,1 - 1,3 ppm (15 H) massif C- $(CH_2)$ n $CH_3$
1,4 ppm (9 H) singulet $(CH_3)_3$ - C
3,0 ppm (2 H) quadruplet $CH_3$ - N
4,7 ppm (1 H) massif NH - C-
||
O

c/ Préparation du carbonate de chlorométhyle et de tertiobutyle

On procède comme dans l'exemple 5 a/. A partir de 7,4 g (0,1 mole) de tertiobutanol, 15,48 g de chloroformiate de chlorométhyle et 8,1 ml de pyridine, on obtient 9,2 g (55 %) de carbonate de tertiobutyle et de chlorométhyle.

Pt Eb: 82°C/2 kPa (15 mm Hg)

RMN $H^1$: 1,4 ppm $(CH_3)_3$ - C, s
5,8 ppm $CH_2$ - Cl, s

I R: $\nu$C = 0 1750 $cm^{-1}$

d/ Réaction du carbonate de chlorométhyle et de tertiobutyle avec la n-octylamine

On opère comme en 5 b/ mais en remplaçant le carbonate de 1-chloro-éthyle et tertiobutyle par le carbonate de chlorométhyle et tertiobutyle. A partir de 6,5 g de n-octylamine et 8,5 g de carbonate de chlorométhyle et tertiobutyle, on obtient 4,3 g (38 %) de carbamate de tertiobutyle et n-octyle identique au précédent (5 b/).

**Exemple 6** - Préparation du N-n-octylcarbamate de furfuryle

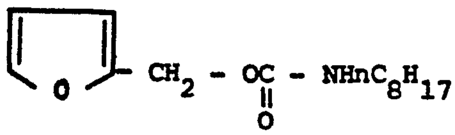

a/ Synthèse du carbonate d'α-chloroéthyle et de furfuryle

On opère comme à l'exemple 5 a/ mais en introduisant goutte à goutte 0,22 mole de chloroformiate d'α-chloroéthyle dans une solution de 0,2 mole d'alcool furfurylique et 0,24 mole de pyridine dans 200 ml de dichlorométhane.

On recueille 35,55 g (rendement 87 %) de carbonate d'α-chloroéthyle et de furfuryle.

Pt Eb: 94-98° C/13,3 Pa (0,1 mm Hg)

I R: $\nu$C = 0 1750 $cm^{-1}$

RMN $H^1$: 1,8 ppm (3 H) doublet $CH_3$ - C
5,2 ppm (2 H) singulet $CH_2O$
6,3 - 6,6 ppm (3 H) massif H - C = et H - 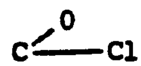

7,5 ppm (1 H) massif H 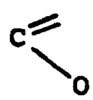

b/ Réaction du carbonate d'α-chloroéthyle et de furfuryle avec la n-octylamine

On opère comme à l'exemple 5 b/ mais avec 10,2 g (0,05 mole) du carbonate précédent dans 30 ml de THF et 12,9 g (0,1 mole) de n-octylamine dans 15 ml de THF.

On obtient 11,05 g (rendement 87 %) du carbamate attendu.

Pt Eb: 162° C/66,6 Pa (0,5 mm Hg), Pt fusion (Pt F): 29° C

I R: $\nu$C = 0 1700 $cm^{-1}$
$\nu$NH 3340 $cm^{-1}$

RMN $H^1$: 0,7 a 1,5 ppm (15 H) massif $(CH_2)$ n $CH_3$
3,1 ppm (2 H) quadruplet $CH_2N$
4,9 ppm (1 H) massif large NH
5,0 ppm (2 H) singulet $CH_2O$
6,4 ppm (2 H) massif H - C =
7,4 ppm (1 H) massif H - 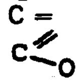

**Exemple 7** - Préparation du N-n-octylcarbamate de furfuryle

Dans un réacteur, on introduit 6,5 g (0,05 mole) de n-octylamine, 30 ml de THF et 20 ml d'une solution aqueuse de $K_2CO_3$ 5 M.

On introduit ensuite goutte à goutte, sous agitation, en maitenant la température à 5 - 10° C, 11,25 g (0,055 mole) de carbonate d'α-chloroéthyle et de furfuryle.

On laisse le mélange revenir à température ambiante et on le maintient sous agitation à cette température pendant 18 heures.

On ajoute 50 ml d'eau saturée en chlorure de sodium, extrait avec 2 fois 40 ml d'éther éthylique, on rassemble et on sèche les phases éthérées sur sulfate de magnésium.

Après élimination du solvant et distillation sous pression réduite, on recueille 9,5 g (75 %) du carbamate attendu.

Pt Eb: 142° C/26,6 Pa (0,2 mm Hg)

**Exemple 8** - Préparation du N-n-octylcarbamate de benzyle

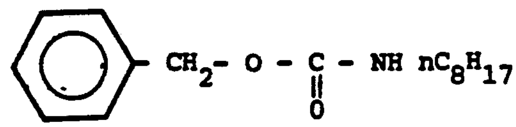

a/ Synthèse de carbonate d'α-chloroéthyle et de benzyle

On opère comme à l'exemple 5 a/ mais avec 200 ml de dichlorométhane, 21,6 g (0,2 mole) d'alcool benzylique, 31,6 g (0,22 mole) de chloroformate d'α-chloroéthyle et 0,2 mole de pyridine.

On recueille ainsi 40,5 g (rendement 94 %) de carbonate d'α-chloroéthyle et de benzyle.

Pt EB: 100° C/66,6 Pa

I R: γC = 0 1760 $cm^{-1}$

RMN $H^1$:
- 1,8 ppm (3 H) doublet $CH_3$-
- 5,2 ppm (2 H) singulet $CH_2$
- 6,4 ppm (1 H) quadruplet O - CH - Cl
- 7,3 ppm (5 H) singulet protons aromatiques

b/ Réaction du carbonate d'α-chloroéthyle et de benzyle avec la n-octylamine

On opère comme à l'exemple 5 b/ mais avec 19,4 g (0,15 mole) de n-octyl-amine dans 30 ml de THF et 16,2 g (0,075 mole) du carbonate précédent dans 40 ml de THF.

On obtient 17,7 g (rendement 90 %) du carbamate attendu.

Pt Eb: 180° C/66,6 Pa (0,5 mm Hg)

Pt F: 33 - 34° C

I R: γC = 0 1680 $cm^{-1}$
γN H 3380 $cm^{-1}$

RMN $H^1$:
- 0,7 à 1,5 ppm (15 H) massif $(CH_2)n$ $CH_3$
- 3,1 ppm (2 H) qadruplet $CH_2N$
- 4,8 ppm (1 H) singulet NH
- 5,1 ppm (2 H) singulet $CH_2$ 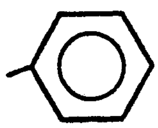
- 7,3 ppm (5 H) singulet protons aromatiques

**Exemple 9** - Préparation du N-isobutylcarbamate de phényle

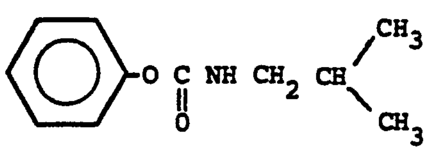

a/ Synthèse du carbonate d'α-chloroéthyle et de phényle

On opère comme à l'exemple 5 a/ mais avec 500 ml de dichlorométhane, 47 g (0,5 mole) de phénol, 79 g (0,55 mole) de chloroformiate d'α-chloroéthyle et 0,5 mole de pyridine.

On recueille ainsi 94,23 g (94 %) de carbonate d'α-chloroéthyle et de phényle.

Pt EB: 110°/66,6 Pa (0,5 mm Hg)

I R: γC = 0 1770 $cm^{-1}$

RMN $H^1$: 1,7 ppm (3 H) doublet $CH_3$
6,35 ppm (1 H) quadruplet CH-Cl
7,0 à 7,3 ppm (5 H) massif protons aromatiques

b/ Réaction du carbonate d'α-chloroéthyle et de phényle avec l'isobutylamine

On opère comme à l'exemple 7 mais avec 7,3 g (0,1 mole) d'isobutylamine, 35 ml d'une solution aqueuse de $K_2CO_3$ 5M et 20,1 g (0,1 mole) du carbonate précédent.
Après élimination du solvant et recristallisation dans l'éther de pétrole, on obient 12,5 g (65 %) du produit attendu.
Pt: 66 - 67 %

I R: γC = 0 1710 $cm^{-1}$
NH: 3400 $cm^{-1}$

RMN $H^1$: 0,9 ppm (6 H) doublet $CH_3$
1,8 ppm (1 H) multiplet 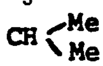
3,1 ppm (2 H) triplet $CH_2$- N
5,3 ppm (1 H) singulet large NH
7,0 à 7,3 (5 H) massif protons aromatiques

**Exemple 10** - Préparation de la tertiobutyloxycarbonyl pipéridine

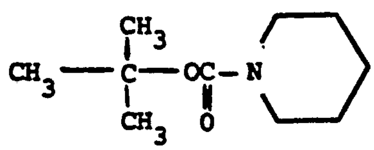

On opère comme à l'exemple 7 mais avec 8,5, g (0,1 mole) de pipéridine, 60 ml de THF, 20 ml d'une solution aqueuse saturée en $K_2$ $CO_3$ et 0,11 mole) de carbonate d'α-chloroéthyle et de tertiobutyle. On agite 2 heures seulement. On recueille 14,8 g (80 %) du carbamate attendu
Pt Eb: 96 - 98°C/2 kPa (15 mm Hg)
I R: γC = 0 1690 $cm^{-1}$

RMH $H^1$: 1,3 - 1,6 ppm (15 H) - $CH_2$ -, $CH_3$ -
3,3 ppm (4 H) $CH_2N$

**Exemple 11** - Préparation de N-n-octylcarbamate de S-éthyle

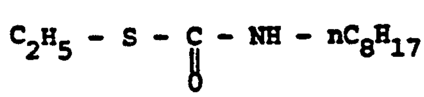

a/ Préparation du thiocarbonate d'α-chlorcéthyle et de S-éthyle

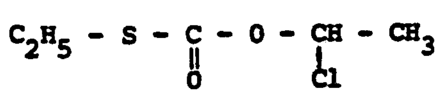

On opère comme à l'exemple 5 a/ mais en introduisant 31,5 g (0,22 mole) de chloroformate d'α-chloroéthyle dans une solution de 12,4 g (0,2 mole) d'éthanéthiol et 15,8 g (0,2 mole) de pyridine dans 200 ml de dichlorométhane.

On obtient 21,1 g (62,5 %) de thiocarbonate d'α-chloroéthyle et de S-éthyle.
Pt Eb: 110°C/5,86 kPa (44 mm Hg)

I R: γC = 0 $1720\ cm^{-1}$

RMN $H^1$: 1,3 ppm triplet $CH_3$
1,75 ppm doublet $CH_3$
2,8 ppm quadruplet $CH_2$ - S
6,5 ppm quadruplet 0 -CHCl

b/ Réaction du triocarbonate d'α-chloroéthyle et de S-éthyle avec la n-octylamine

On opère comme à l'exemple 7 mais avec 6,5 g (0,05 mole) de n-octylamine, 30 ml de THF, 20 ml d'une solution aqueuse de $K_2CO_3$ 5M et 8,43 g (0,05 mole) du thiocarbonate précédent.
On recueille ainsi 5,3 g (49 %) du thiocarbamate attendu.
Pt Eb: 146-152°/66,6 Pa (0,5 mm Hg)

I R: γC = 0 $1650\ cm^{-1}$
γNH $3300\ cm^{-1}$

RMN $H^1$: 0,7 à 1,6 ppm (18 H) massif $(CH_2)nCH_3$
2,8 ppm (2 H) quadruplet $CH_2S$
3,1 ppm (2 H) pseudo triplet $CH_2N$
3,2 ppm (1 H) singulet large NH

**Exemples 12 à 19** Préparation de différents carbamates de benzyle

Dans ces exemples on fait réagir le carbonate d'α-chloroéthyle et de benzyle selon le mode opératoire de l'exemple 7 avec des amines primaires ou secondaires variées.
Les conditions de température et de durée de réaction, les propriétés physiques des produits obtenus et les rendements sont indiqués sur le tableau I.

**Tableau I**

| Ex. n° | Amines utilisées | Temps | Température | Produits obtenus | pt d'ébullition [pt fusion] | rendements en produits purifiés |
|---|---|---|---|---|---|---|
| 12 | 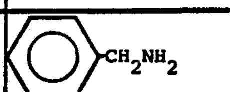 | 5 h | 20°C | 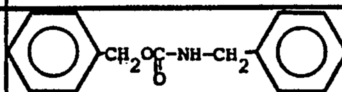 | 175°C/13,3 Pa [64°C] | 84% |
| 13 | 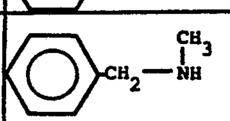 | 4 h | 20°C | 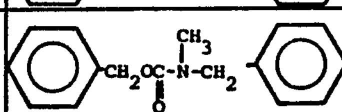 | 165°C/133,3 Pa | 87% |
| 14 | 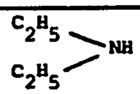 | 2 h | 20°C | 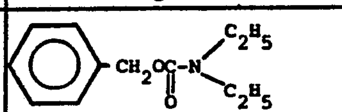 | 96°C/66,7 Pa | 99% |
| 15 | 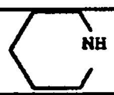 | 2 h | 20°C | 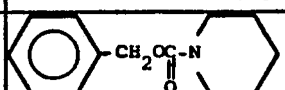 | 128°C/66,7 Pa | 97% |
| 16 | 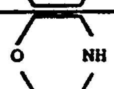 | 3 h | 20°C | 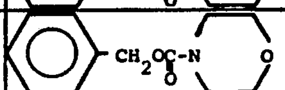 | 140°C/13,3 Pa | 84% |
| 17 | 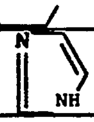 | 18 h | 20°C | 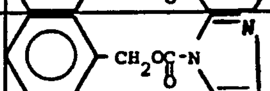 | 130°C/133,3 Pa | 50% |
| 18 | $NH_2-CH_2CH_2OH$ | 5 h | 20°C | 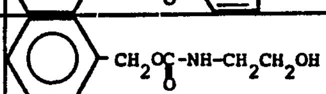 | 170°C/66,7 Pa | 74% |
| 19 | 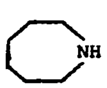 | 2 h | 20°C | 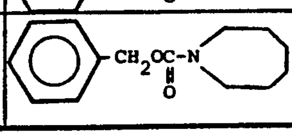 | 165°C/66,7 Pa | 87% |

**Exemple 20** - Préparation du N'N-hexaméthylène thiocarbamate de S-éthyle - (MOLINATE)

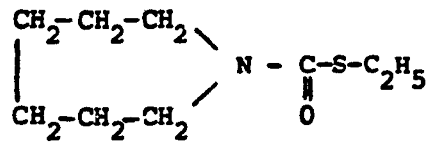

On opère comme à l'exemple 11 par réaction du thiocarbonate d'α-chloroéthyle et de S-éthyle avec l'hexaméthylène imine.

On obtient le "MOLINATE" avec un rendement de 70 % en produit distillé (Pt Eb: 141° C/1,7 kPa (13 mm Hg)

**Exemple 21** - Préparation du N-benzylcarbamate de tertio-butyle

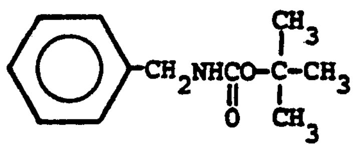

a/ Synthèse du carbonate de tétrachloro-1,2, 2,2 éthyle et de tertio-butyle.

On ajoute en une seule fois 9,9 g (0,04 mole) de chloroformiate de tétrachloro-1,2,2,2 éthyle à une solution de tertiobutanol (3 g; 0,04 mole) dans le dichlorométhane (50 ml). On refroidit à 0° C et on ajoute goutte à goutte 3,2 g (0,04 mole) de pyridine. On agite 4 heures à température ambiante. On ajoute alors 20 ml d'eau glacée, on sépare la phase organique et on lave avec 20 ml d'eau glacée. On sèche sur sulfate de magnésium et on évapore le solvant. On obtient 11,3 g d'un solide blanc (rendement: 99 %) que l'on recristallise dans l'éther de pétrole (rendement 87 %; Pt F: 70° C), et l'on obtient 9,9 g du carbonate purifiée.

Pt Eb: 96° C/866 Pa (6,5 mm Hg)

I R: $\nu C = 0$ 1770 $cm^{-1}$

RMN $H^1$ ($CDCl_3$, TMS): 1,5 (s, $CH_s$) 6,7 (s, CH)

b/ Réaction du carbonate précédent avec la benzylamine

On dissout 1,1 g (0,01 mole) de benzylamine dans 20 ml de THF et on ajoute 3 ml de carbonate de potassium 5 M en solution aqueuse.

On ajoute alors à 5° C 2,9 g (0,01 mole) de corbonate de tertiobutyle et tétrachloréthyle en solution dans 5 ml de THF. On agite une heure à 20° C, on décante la phase organique, on lave avec 10 ml d'une solution aqueuse saturée en NaCl. On sèche la phase organique, on évapore et on distille: on obtient 2,0 g du carbamate attendu (rendement: 96 %); Pt Eb: 103° C/6,7 Pa (0,05 mm Hg).

On recristallise dans l'éther de pétrole et on obtient 1,84 g du carbamate attendu (89 %); Pt F = 54° C (Litt = 53-54° C)

**Exemple 22** - Prépration du tertiobutyloxcarbonylimidazole

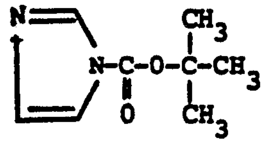

On ajoute à 0° C 5 g (17,5 mmole) de carbonate de tertiobutyle et de tétrachloro-1,2,2,2 éthyle dissout dans 10 ml de THF à une solution d'imidazole (1,2 g; 17,6 mmoles) dans le THF (20 ml) en présence d'une solution aqueuse 5 M (5 ml) de carbonate de potassium. On agite une heure à 20° C, on décante et on lave avec 10 ml

d'une solution aquenuse saturée en NaCl. Apré séchage et évaporation des solvants on distille le résidu obtenu, on obtient: 2,55 g (rendement 86 %)

Pt Eb: 64°C/133,3 Pa (1 mm Hg)

Pt F: 43°C

**Exemple 23** - Preparation du (2,3-dihydro-2,2-diméthyl-7-benzofuranyl)-

N-méthyl carbamate (CARBOFURAN)

a/ Synthèse du carbonate de 1-chloro-éthyle et 2,3-dihydro-2,2 diméthyl-7-benzofuranyle.

On ajoute en une seule fois 21,5 g (0,15 mole) de chloroformiate de 1-chloroéthyle à une solution de 2, 3-dihydro-2, 2-diméthyl-7-benzofu-ranol (24,6 g; 0,15 mole) dans le dichlorométhane (150 ml). On refroidit entre 0 et 5°C et on ajoute goutte à goutte 12 g (0,15 mole) de pyridine. On agite 3 heures à 20°C. On lave alors la phase organique avec 2 x 50 ml d'eau glacée. On sèche sur sulfate de magnésium et on évapore le solvant. On obtient une huile jaune que l'on distille. On récupère alors 34,1 g du carbonate attendu (rendement 84 %).

Pt Eb = 127°C/66,6 Pa (0,5 mm Hg).

b/ Réaction du carbonate précédent avec la méthylamine

On dissout 5,4 g (0,02 mole) du carbonate précédent dans le THF (20 ml). On ajoute alors 10 ml d'une solution aqeuse de $K_2CO_3$ environ 5 M puis 1,7 ml (0,02 mole) d'une solution de méthylamine à 40 % dans l'eau. On agite 15 heures à 20°C. On décante et on lave la phase organique avec une solution saturée de NaCl. On évapore le solvant et on cristallise dans le méthylcyclohexane. On obtient 3,5 g du carbamate souhaité (rendement 79 %).

Pt F = 148°C.

c/ On opère comme en a/ puis b/ mais en remplaçant la pyridine par la N,N-diméthylaniline et en ne distillant pas le carbonate intermédiaire. A partir de 16,4 g de 2,3-dihydro-2,2-diméthyl-7-benzofuranol on obtient alors 16,8 g (76 %) de CARBOFURAN. Pt F = 147°C.

**Exemple 24** - Préparation de la N-méthyl, N'-pipéridinylurée

a/ Synthèse du N-méthyl carbamate de tétrachloro-1,2,2,2 éthyle.

On ajoute goutte à goutte 34,7 ml (0,4 mmole) de méthylamine (en solution aqueuse à 40 %) à une solution maintenue à 0°C de 49,4 g (0,2 mole) de chloroformiate de tétrachloro-1,2,2,2 éthyle dans le dichlorométhane (150 ml). On agite ensuite deux heures à température ambiante. On lave la phase organique avec 2 x 100 ml d'eau, on sèche sur sulfate de magnésium.

Le produit cristallise par évaporation du solvant, on obtient 42,6 g du carbamate attendu (rendement 88 %)

Pt F = 105 - 106°C

RMN $H^1$: 2,75 ($CH_3$-N
5,2 (NH)
6,7 (CH-Cl)

IR: $\gamma C=O$ 1760 $cm^{-1}$

b/ Synthèse de la N-méthyl, N'-pipéridinylurée

On dissout 4,83 g (0,02 mole) du carbamate précédemment obtenu dans 30 ml de THF et 5 ml d'eau saturée avec $K_2CO_3$. On ajoute 1,7 g (0,02 mole) de pipéridine à cette solution maintenue à 10°C et on agite 4 heures à température ambiante. On décante et on lave la phase organique avec (50 ml d'eau saturée de Na Cl. On sèche sur sulfate de magnésium,on évapore et on distille le résidu. On obtient 1,8 g d'urée (rendement 63 %).

Pt Eb: 110°C/6,7 Pa (0,05 mm Hg)

**Exemple 25** - Préparation de l'imidazolylcarbonylpipéridine

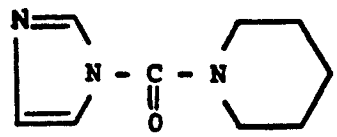

a/ Synthèse de l'α -chloroéthoxycarbonylimidazole

On ajoute goutte à goutte 28,6 g (0,2 mole) de chloroformiate d'- chloroéthyle à une solution de 27,2 g (0,4 mole) d'imidazole dans 200 ml de dichlorométhane refroidie par un bain d'eau.

On agite à température ambiante pendant 4 heures puis on ajoute 50 ml d'eau glacée. La phase organique est lavée par 2 x 50 ml d'eau puis séchée sur sulfate de magnésium. Après évaporation et distillation, on obtient 38,1 g (73 %) d'α(-chloroéthoxycarbonyl imidazole Pt Eb: 80°C/66,6 Pa (0,5 mm Hg) sous forme d'un liquide incolore qui cristallise spontanément à température ambiante Pt F: 50°C

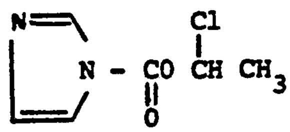

RMN $H^1$ (60 MHz, $CDCl_3$, TMS) δ: 1,9 (d, $CH_3$)
6,7 (q, OCH-Cl)
7,05; 7,4; 8,2 (3 pesudosingulets imidazole)

I R: $\gamma$C = 0 1770 $cm^{-1}$

b/ Réaction de l'α-chloroéthoxycarbonyl imidazole avec la pipéridine

On ajoute goutte à goutte une solution de 8,5 g (0,1 mole) de pipéridine dans 10 ml de THF a une solution d'α-chloroéthoxycarbonylimidazole (8,75 g; 0,05 mole) dans 50 ml de THF.

Cette solution est refroidie à +5°C pendant la durée de l'addition puis agitée à température ambiante.

On filtre le chlorhydrate de pipéridine formé puis on lave une fois à l'eau la phase organique.

Après séchage sur sulfate de magnésium et évaporation du solvant, on distille et on récupère ainsi 6,2 g (rendement: 70 %) du produit attendu.

Pt Eb: 134°C/26,6 Pa (0,2 mm Hg)

Le liquide obtenu cristallise spontanément au réfrigérateur.

Pt F: 38°C

RMN $H^1$: 1,5 ppm multiplet $(CH_2)_3$
3,5 ppm multiplet $CH_2$- 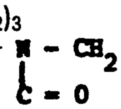

7,0 ppm imidazole
7,15 ppm imidazole
7,8 ppm imidazole

I R: $\gamma$C = 0 1690 $cm^{-1}$

**Exemple 26** - Préparation de la N,N-diéthyl imidazole carboxamide

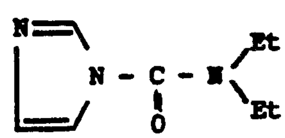

On opère comme à l'exemple 25 b/ mais en remplaçant la pipéridine par la diéthylamine. On obtient 6,5 g de l'urée souhaitée (rendement 78 %).

Pt Eb: 106° C/26,6 Pa (0,2 mm Hg)
Pt F: 41° C (litt. 38-43° C)

RMN $H^1$: 1,2 ppm (t, $CH_3$)
3,4 ppm (q, $CH_2N$)
7,0 ppm imidazole
7,2 ppm imidazole
7,8 ppm imidazole

I R: $\nu$C = 0 1690 $cm^{-1}$

**Exemple 27** - Préparation de méthyl-2 (méthylthio)-2 propanal O- [(méthylamino) carbonyl] oxime (ALDICARBE)

On ajoute successivement 50 ml de toluène et 6,65 g (0,05 mole) de 2-méthyl-2 (méthylthio) propanal oxime à 5 ml de soude 10 N. On agite quelques instants à température ambiante puis on enlève l'eau par distillation azéotropique. On refroidit ensuite dans un bain d'eau glacée et on ajoute goutte à goutte 7,15 g (0, 05 mole) de chloroformiate de 1-chloroéthyle. On agite une heure à environ 10 - 15° C. Pour obtenir le carbonate de méthyl-2 (méthylthio)-2 propanal oxime et de chloro-1 éthyle, on ajoute alors goutte à goutte 10 ml (environ 0,13 mole) de méthylamine aqueuse à 40 % et on agite encore une heure à la même température. On décante et on lave la phase organique avec 10 ml d'eau glacée. On sèche la phase organique sur sulfate de magnésium et on évapore à sec. On obtient 8,7 g de solide légèrement brun Pt F = 82 - 90° C (70 % pur par HPLC) qui peut être purifié par chromatographie sur colonne de gel de silice ou par cristallisation dans l'éther isopropylique (rendement 63 %, Pt F = 98 - 100° C). RMN$H^1$ ($CDCl_3$ 60 MHz) 1,42 ppm (s,6 H) 1,92 (s,3 H) 2,92 (d,3H) 7,54 (s,1H).

**Exemple 28** - Synthèse de l'acide tertiobutyloxycarbonyl-L-aspartique

A une solution d'acide L-aspartique 1,33 g (10 mmoles) dans le dioxanne aqueux (1: 1; 30 ml) on ajoute 4,2 ml (30 mmoles) de triéthylamine et on agite jusqu' à dissolution (environ 10 min). On ajoute alors 2,85 g (10 mmoles) de carbonate de tertiobutyle et de tétra-chloro-1,2,2,2 éthyle et on agite 6 heures à 20° C. On ajoute alors 50 ml d'eau et on extrait avec 2 x 20 ml d'acétate d'éthyle. La phase aqueuse est acidifiée (pH 2-3) avec HCl N puis extraite avec 3 x 30 ml d'acétate d'éthyle. L'extrait est lavé avec une solution saturée de NaCl, séché sur $MgSO_4$ et évaporé. Le produit obtenu est cristallisé dans l'acétate d'éthyle et l'éther de pétrole. On obtient 1,4 g (rendement 60 %) de l'acide attendu.

Pt F = 116 - 118°C        Pt $F_{Litt}$ = 114 - 116°C

$[\alpha]_D^{20}$ = - 5 (c 1,0 MeOH)        $[\alpha]_{D\ Litt}^{20}$ = - 6,2 (c 1,0 MeOH)

**Exemple 29** - Préparation du furfuryloxycarbonyl-glycinate d'éthyle

On ajoute 2,05 g (10 mmoles) du carbonate d'α-chloroéthyle et de furfuryle à une solution de 1,03 g (10 mmoles) de glycinate d'éthyle dans 6 ml de THF et 4 ml d'une solution de carbonate de potassium 0,5 M maintenue entre 5 et 10° C. On laisse revenir à température ambiante et on agite 18 heures. On ajoute 50 ml d'eau saturée en NaCl et on extrait avec 3 fois 40 ml d'éther éthylique. On rassemble et on séche les phases organiques sur sulfate de magnésium. Après élimination du solvant et distillation, on recueille 1,5 g (rendement 66 %) du produit attendu.

Pt Eb: 144° C/40 Pa (0,3 mm Hg)

I R: $\nu$C = 0 1680 $cm^1$
$\nu$NH 3280 $cm^1$

RMN $H^1$ ($CDCl_3$ TMS): 1,3 ppm triplet $CH_3$

3,95 ppm doublet 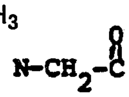

4,2 ppm quadruplet $CH_2$ ($CH_3$)

5,1 ppm signulet 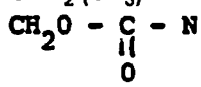

5,2 ppm singulet large NH

6,4 ppm massif H-C=

7,4 ppm massif 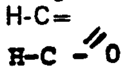

**Exemple 30** - Préparation de la benzyloxycarbonyl-L-proline

A une solution de 1,15 g (10 mmoles) de L-proline dans 10 ml de méthanol et 3 ml de $K_2CO_3$ aqueux saturé, on ajoute à 5°C, 2,36 g (11 mmoles) de carbonate de benzyle et a -chloroéthyle. Après 4 heures de réaction, on ajoute 50 ml d'eau et on lave avec 2 fois 10 ml d'éther éthylique. On acidifie à PH 2-3 avec HCl 6 N et on extrait à l'acétate d'éthyle.

Après évaporation des solvants et cristallisation dans le mélange acétate d'éthyle-éther de pétrole on obtient 2,2 g (rendement 88 %) de Z-(L)-pro;

Pt F: 75-76°C (Pt $F_{Litt}$ = 76-78°C)

**Exemple 31** - Préparation du carbonate de tétrachloro-1,2,2,2 éhyle et 2-triméthyl silyléthyle.

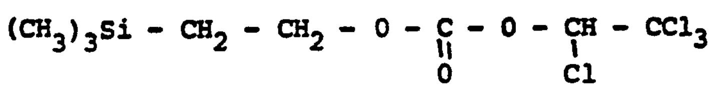

On procède comme dans l'exemple 21 a). A partir de 5,91 g de triméthyl silyléthanol et 12,35 g de chloroformiate de tétrachloroéthyle on obtient 13,6 g (rendement 83 %) de produit attendu;

E = 92-94°C/6,6 Pa

I R $\gamma$CO = 1750 $cm^{-1}$

RMN $H^1$ ($CDCl_3$, TMS externe) = 0,1 (s, $CH_3$-Si) 1,1 (t, $CH_2$-Si) 4,35(t, $CH_2$-O) 6,7(s, CH-Cl)

**Exemple 32** - Préparation de la triméthylsilyléthyloxycarbonyl-(L) -phénylanine

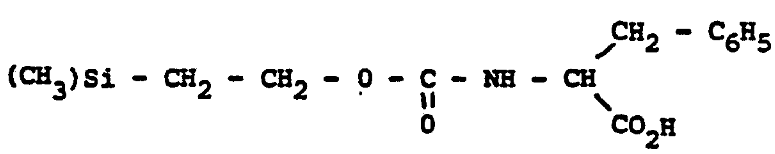

On dissout 0,83 g de L-phenylalanine (5 mmoles) dans le dioxanne aqueux (1: 2; 12 ml) contenant 1,4 ml de triéthylamine (10 mmoles). On refroidit à 0°C et on ajoute en une fois 1,8 g (5,5 mmoles) du carbonate précédent dissout dans 4 ml de dioxanne. Après 2 heures à 0°C on ajoute 20 ml d'eau et on extrait avec 2 fois 20 ml d'éther. On acidifie alors la phase aqueuse (PH-2-3) avec HCl 6N et on extrait avec 3 fois 50 ml d'acétate d'éthyle. On sèche sur $MgSO_4$ et on évapore. On obtient 1,4 g (rendement 100 %) du produit attendu sous

forme d'une huile.

RMN $H^1$ ($CDCl_3$, TMS) O(s,$CH_3$-Si) 0,9(t, $CH_2$ -Si) 3,0($CH_2$ Ph)

4,0(t,O-$CH_2$-C-Si) 4,5(m,CH($CO_2$) - N) 5,2(s,NH) 7,2(s,Ph) 8,7(C(=O)-OH)

On ajoute 2 ml de dicyclohexylamine à cette huile dissoute dans 5 ml d'éther et on recueille après cristallisation 1,93 g (rendement 78 %) de sel de dicyclohexylammonium Pt F = 111-112°C.

## Revendications

1. Procéde de preparation de derives de l'acide carbonique de formule:

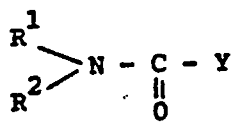

dans laquelle $R^1$, $R^2$ identiques ou differents représentent:
- un atome d'hydrogène,
- un radical aliphatique ou araliphatique, linéaire ou ramifié, saturé ou insaturé, substitué ou non,
- un radical cycloaliphatique saturé ou insaturé, substitué ou non,
- un radical heterocyclique saturé ou non, substitué ou non, ou forment ensemble avec l'atome d'azote auquels ils sont liés un cycle substitué ou non, saturé ou non, qui peut comporter un ou plusieurs autres hétéroatomes et qui peut faire partie d'un système cyclique,

Y représente les groups OR, SR,

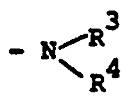 ou 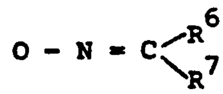

dans lesquels R représente un radical aliphatique ou araliphatique, linéaire ou ramifié, saturé ou insaturé, substitué ou non, un reste cycloaliphatique saturé ou insaturé, substitué ou non, un reste aromatique substitué ou non, $R^3$ et $R^4$ identiques ou différents représentent un atome d'hydrogène, un radical aliphatique, araliphatique, cycloaliphatique ou hétérocyclique, saturé ou non, substitué ou non, un radical aromatique substitué ou non, ou forment ensemble avec l'atome d'azote auquel ils sont liés, un hétérocycle, pouvant comporter un ou plusieurs autres hétéroatomes, substitué ou non, saturé ou non et $R^6$ et $R^7$ identiques ou différents représentent un radical aliphatique ou cycloaliphatique, linéaire ou ramifié, substitué ou non, saturé ou non, ou représentent mais pas en même temps, un atome d'hydrogène, un radical alkylthio, un radical alkyloxy, caractérisé en ce que l'on fait réagir en présence d'un accepteur d'acide halohydrique, à une température comprise entre -5 ° et 150°, un composé aminé hydrogéné de formule:

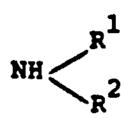

avec un dérivé α-halogéné de l'acide carbonique de formule:

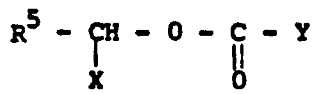

dans lesquelles $R^1$, $R^2$ et Y ont les significations précédentes, X représente un atome de fluor, chlore ou brome et $R^5$ représente un atome d'hydrogène, un reste aliphatique, araliphatique ou cycloaliphatique, saturé ou insaturé, substitué ou non, un reste aromatique substitué ou non.

2. Procédé selon la revendication 1 caractérisé en ce que X représente un atome de chlore.

3. Procédé selon la revendication 1 ou 2, caractérisé en ce que le radical $R^5$ est un radical aliphatique comportant 1 à 4 atomes de carbone substitué ou non par des atomes d'halogène.

4. Procéde selon la revendication 1, 2 ou 3 caractérisé en ce que le radical R est un radical aliphatique comportant de 1 à 12 atomes de carbone, un radical furfuryle, benzyle, phényle, substitué ou non, et les radicaux $R^3$ et $R^4$ représentent un atome d'hydrogéne, le radical méthyle, ou forment ensemble avec l'atome d'azote un cycle imidazolyle.

5. Procédé selon la revendication 1, 2 ou 3, caractérisé en ce que R est un des groupes protecteurs de la fonction amine des aminoacides, habituellement utilisé dans la synthèse peptidique.

6. Procédé selon l'une quelconque des revendications précédentes caractérisé en ce que $R^6$ et $R^7$, indentiques ou différents, représentent un radical aliphatique en $C_1$ à $C_{12}$ ou cycloaliphatique pouvant comporter 30 atomes de carbone, substitué ou non par des groupes comportant du soufre et, pas tous les deux en même temps, un atome d'hydrogène, un radical méthylthio.

7. Procédé selon la revendication 4, 5 ou 6, caractérisé en ce que le dérivé α-halogéné est le carbonate d'α-chloroéthyle et d'éthyle ou de tertiobutyle, de furfuryle, de benzyle, de phényle ou de 2,3-dihydro-2, 2 diméthyl-7 benzofuranyle, le carbonate de tétrachloro-1,2,2,2 éthyle et de tertiobutyle, le thiocarbonate d'α-chloroéthyle et de S-éthyle, l'α-chloroéthoxyimidazole, le N-méthylcarbamate de tétrachloro-1,2,2,2 éthyle, le carbonate de tétrachloro-1,2,2,2 éthyle et de 2-triméthylsilyléthyle, le carbonate d'α-chloroéthyle et de méthyl-2 (méthylthio)2 propanaloxime.

8. Procédé selon l'une quelconque des revendications précédentes caracterise en ce que les radicaux $R^1$ et $R^2$ du composé aminé, identiques ou différents représentent un atome d'hydrogène, un radical aaliphatique comportant de 1 à 20 atomes de carbone, cycloaliphatique ou araliphatique comportant jusqu' à 50 atomes de carbone, ou forment ensemble avec l'atome d'azote un cycle piperidino, morpholino, imidazolyle, le radical ou le cycle étant substitué ou non par des groupes acide, alcool, ester, éther, mercapto, amino.

9. Procédé selon l'une quelconque des revendications précédentes, caracterise en ce que $R^1$ et $R^2$ sont les radicaux habituels des acides aminés naturels ou synthétiques.

10. Procédé selon l'une quelconque des revendications précédentes, caractérisé en ce que l'amine est la méthylamine, la diéthylamine, la di n-butylamine, l'isobutylamine, la n-octylamine, l'éthanolamine, la benzylamine, l'imldazole, l'hexaméthylène-imine, la morpholine, la diéthanolamine, la N-méthyl N-benzylamine, la pipéridine, la L-phénylalanine, la L-proline, la glycine, la L-tyrosine, la L-serine, l'acide L-aspartique, le glycinate d'éthyle, la phénylglycine, la proline.

11. Procédé selon l'une quelconque des revendications précédentes caractérisé en ce que la réaction est effectuée en présence d'un ou de plusieurs solvants inertes vis-à-vis des réactifs.

12. Procédé selon l'une quelconque les revendications précedéntes caractérisé en ce que les solvants sont choisis parmi les solvants aliphatiques chlorés, les éthers cycliques ou non, les alcools, l'acétone, la pyridine, l'acétonitrile ou le diméthylforuamide.

13. Procede selon les revendication 11 ou 12 caractérisé en ce que le milieu solvant contient de l'eau.

14. Procédé selon l'une quelconque des revendications précédentes caractérisé en ce que l'accepteur d'acide est une base organique ou minérale.

15. Procédé selon l'une quelconque des revendications précédentes caractérisé en ce que l'accepteur d'acide est l'hydroxyde de sodium ou de potassium, le sulfite de sodium, le carbonate ou bicarbonate de sodium ou de potassium, l'oxyde de magnésium, une amine tertiaire ou l'amine de départ de formule

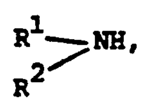

$R^1$ et $R^2$ ayant les significations precedentes.

16. Procédé selon l'une quelconque des revendications précédentes caractérisé en ce que l'amine tertiaire est la triéthylamine, la pyridine, ou la N,N-diméthylaniline.

**Patentansprüche**

1. Verfahren zur Herstellung von Carbaminsäurederivaten der Formel

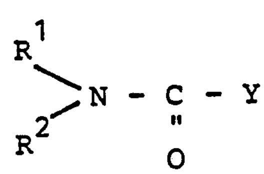

in der bedeuten:

$R^1$ $R^22$ gleich oder verschieden, Wasserstoff, einen gegebenenfalls substituierten, gesättigten oder ungesättigten, geradkettigen oder verzweigten aliphatischen oder arylaliphatischen Rest, einen gegebenenfalls substituierten, gesättigten oder ungesättigten cycloaliphatischen Rest, einen gegebenenfalls substituierten, gesättigten oder ungesättigten heterocyclischen Rest oder zusammen mit dem Stickstoffatom, an das sie gebunden sind, einen gegebenenfalls substituierten, gesättigten oder ungesättigten Ring mit einem oder mehreren anderen Heteroatomen bilden, der Teil eines Ringsystems sein kann,

Y OR, SR, 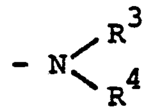 oder 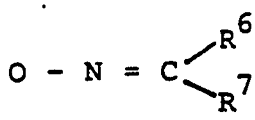 ,

wobei R ein gegebenenfalls substituierter, gesättigter oder ungesättigter, geradkettiger oder verzweigter aliphatischer oder arylaliphatischer Rest, ein gegebenenfalls substituierter, gesättigter oder ungesättigter cycloaliphatischer Rest, ein gegebenenfalls substituierter aromatischer Rest ist,

$R^3$ und $R^4$, gleich oder verschieden, Wasserstoff, einen
550-Regel 51 B 782 EP - MK/TH gegebenenfalls substituierten, gesättigten oder ungesättigten aliphatischen, arylaliphatischen, cycloaliphatischen oder heterocyclischen Rest, einen gegebenenfalls substituierten aromatischen Rest bedeuten oder zusammen mit dem Stickstoffatom, an das sie gebunden sind, einen gegebenenfalls substituierten, gesättigten oder ungesättigten Heterocyclus mit gegebenenfalls einem oder mehreren anderen Heteroatomen bilden und

$R^6$ und $R^7$, gleich oder verschieden, einen gegebenenfalls substituierten, gesättigten oder ungesättigten, geradkettigen oder verzweigten aliphatischen oder cycloaliphatischen Rest oder, jedoch nicht gleichzeitig, Wasserstoff, Alkylthio oder Alkyloxy bedeuten,

gekennzeichnet durch

die Umsetzung eines Amins der Formel

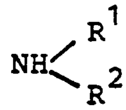

mit einem Carbonsäure-α-halogenderivat der Formel

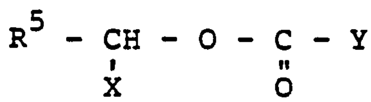

wobei bedeuten:

$R^1$, $R^2$ und Y wie oben angegeben,

X Fluor, Chlor oder Brom und

$R^5$ Wasserstoff, einen gegebenenfalls substituierten, gesättigten oder ungesättigten aliphatischen, arylaliphatischen oder cycloaliphatischen oder einen gegebenenfalls substituierten aromatischen Rest,

in Gegenwart eines Halogenwasserstoffsäure-Akzeptors bei einer Temperatur von -5 bis 150°C.

2. Verfahren nach Anspruch 1, gekennzeichnet durch Verwendung einer Verbindung der oben angegebenen Formel, in der X Chlor ist.

3. Verfahren nach Anspruch 1 oder 2, gekennzeichnet durch Verwendung einer Verbindung der oben angegebenen Formel, in der $R^5$ ein gegebenenfalls mit Halogen subsituierter aliphatischer Rest mit 1 bis 4 Kohlenstoffatomen ist.

4. Verfahren nach Anspruch 1, 2 oder 3, gekennzeichnet durch Verwendung einer Verbindung der oben angegebenen Formel, in der R ein aliphatischer Rest mit 1 bis 12 Kohlenstoffatomen, gegebenenfalls substituiertes Furfuryl, Benzyl oder Phenyl und $R^3$ und $R^4$ Wasserstoff, Methyl sind oder zusammen mit dem Stickstoffatom einen Imidazolylring bilden.

5. Verfahren nach Anspruch 1, 2 oder 3, gekennzeichnet durch Verwendung einer Verbindung der oben angegebenen Formel, in der R eine in der Peptidsynthese herkömmlicherweise verwendete Aminoschutzgruppe für Aminosäuren ist.

6. Verfahren nach einem der vorstehenden Ansprüche, gekennzeichnet durch Verwendung einer Verbindung der oben angegebenen Formel, in der $R^6$ und $R^7$, gleich oder verschieden, einen gegebenenfalls mit schwefelhaltigen Gruppen substituierten aliphatischen Rest mit 1 bis 12 Kohlenstoffatomen oder cycloaliphatischen Rest mit bis zu 30 Kohlenstoffatomen oder, jedoch nicht gleichzeitig, Wasserstoff oder Methylthio bedeuten.

7. Verfahren nach Anspruch 4, 5 oder 6, gekennzeichnet durch Verwendung von α-Chlorethyl-ethyl, -tert.-butyl, -furfuryl, -benzyl, -phenyl-oder 2,3-Dihydro-2,2-dimethyl-7-benzofuranylcarbonat, 1,2,2,2-Tetrachlorethyl-tert.butylcarbonat, α-Chlorethyl-S-ethyl-thiocarbonat, α-Chlorethoxyimidazol, 1,2,2,2-Tetrachlorethyl-N-methylcarbamat, 1,2,2,2-Tetrachlorethyl-2-trimethylsilylethyl-carbonat, α-Chlorethyl-methyl-2-(methylthio)-2-propanal-oximcarbonat als α-Halogenderivat.

8. Verfahren nach einem der vorstehenden Ansprüche, gekennzeichnet durch Verwendung eines Amins der vorstehenden Formel, in der $R^1$ und $R^2$, gleich oder verschieden, Wasserstoff, einen aliphatischen Rest mit 1 bis 20 Kohlenstoffatomen, einen cycloaliphatischen oder arylaliphatischen Rest mit bis zu 50 Kohlenstoffatomen bedeuten oder zusammen mit dem Stickstoffatom einen Piperidino-, Morpholino- oder

Imidazolylring bilden, wobei der Rest oder der Ring gegebenenfalls mit Säure-, Alkohol-, Ester-, Ether-, Mercapto- oder Aminogruppen substituiert ist.

9. Verfahren nach einem der vorstehenden Ansprüche, gekennzeichnet durch Verwendung eines Amins der oben angegebenen Formel, in der $R^1$ und $R^2$ herkömmliche Reste der natürlichen oder synthetischen Aminosäuren sind.

10. Verfahren nach einem der vorstehenden Ansprüche, gekennzeichnet durch Verwendung von Methylamin, Diethylamin, Di-n-butylamin, Isobutylamin, n-Octylamin, Ethanolamin, Benzylamin, Imidazol, Hexamethylenimin, Morpholin, Diethanolamin, N-Methyl-N-benzylamin, Piperidin, L-Phenylalanin, L-Prolin, Glycin, L-Tyrosin, L-Serin, L-Asparaginsäure, Ethylglycinat, Phenylglycin oder Prolin als Amin.

11. Verfahren nach einem der vorstehenden Ansprüche, gekennzeichnet durch die Durchführung der Reaktion in Gegenwart mindestens eines gegenüber den Reaktionsteilnehmern inerten Lösungsmittels.

12. Verfahren nach einem der vorstehenden Ansprüche, gekennzeichnet durch Verwendung von chlorierten aliphatischen Lösungsmitteln, gegebenenfalls cyclischen Ethern, Alkoholen, Aceton, Pyridin, Acetonitril oder Dimethylformamid als Lösungsmittel.

13. Verfahren nach Anspruch 11 oder 12, gekennzeichnet durch Verwendung eines Wasserenthaltenden Lösungsmittels.

14. Verfahren nach einem der vorstehenden Ansprüche, gekennzeichnet durch Verwendung einer organischen oder anorganischen Base als Säureakzeptor.

15. Verfahren nach einem der vorstehenden Ansprüche, gekennzeichnet durch Verwendung von Natrium- oder Kaliumhydroxid, Natriumsulfit, Natrium- oder Kaliumcarbonat oder -hydrogencarbonat, Magnesiumoxid, eines tertiären Amins oder des Ausgangsamins der Formel

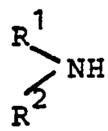

wobei $R^1$ und $R^2$ die oben angegebenen Bedeutungen haben, als Säureakzeptor.

16. Verfahren nach einem der vorstehenden Ansprüche, gekennzeichnet durch Verwendung von Triethylamin, Pyridin oder N,N-Dimethylanilin als tertiäres Amin.

**Claims**

1. Process for preparing carbamic derivatives of formula:

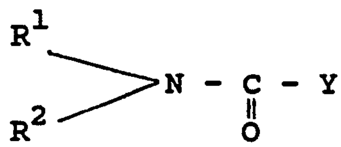

in which $R^1$ and $R^2$, which may be identical or different, denote:
- a hydrogen atom,
- a substituted, saturated or unsaturated, linear or branched aliphtic or araliphatic radical,
- a substituted or unsubstituted, saturated or unsaturated heterocyclic radical, or together form with the nitrogen atom to which they are bound a substituted or unsubstituted, saturated or unsaturated ring which can contain one or more other hetero atoms and which can form part of a ring system,

Y denotes OR, SR,

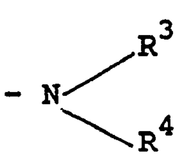 or 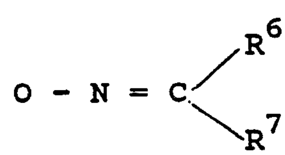 groups

in which R denotes a substituted or unsubstituted, saturated or unsaturated, linear or branched aliphatic or araliphatic radical, a substituted or unsubstituted, saturated or unsaturated cycloaliphatic residue, or a substituted or unsubstituted aromatic residue, $R^3$ and $R^4$, which may be identical or different, denote a hydrogen atom, a substituted or unsubstituted, saturated or unsaturated aliphatic, araliphatic, cycloaliphatic or heterocyclic radical, or a substituted or unsubstituted aromatic radical, or together form with the nitrogen atom to which they are bound a saturated or unsaturated, substituted or unsubstituted heterocycle which can contain one or more other hetero atoms, and $R^6$ and $R^7$, which may be identical or different, denote a saturated or unsaturated, substituted or unsubstituted, linear or branched aliphatic or cycloaliphatic radical, or denote, but not at the same time, a hydrogen atom, an alkylthio radical or an alkyloxy radical, characterised in that a hydrogen-containing amino compound of formula:

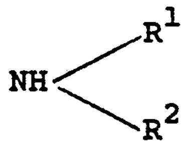

is reacted in the presence of an acceptor for hydrohalic acid, at a temperature between -5°C and 150°C, with an halogenated derivative of carbonic acid of formula:

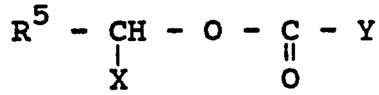

in which $R^1$, $R^2$ and Y have the above significance, X denotes a fluorine, chlorine or bromine atom and $R^5$ denotes a hydrogen atom, a substituted or unsubstituted, saturated or unsaturated, aliphtic, araliphatic or cycloaliphatic residue or a substituted or unsubstituted aromatic residue.

2. Process according to Claim 1, characterised in that X denotes a chlorine atom.

3. Process according to Claim 1 or 2, characterised in that the radical $R^5$ is an aliphatic radical which contains 1 to 4 carbon atoms and is substituted or unsubstituted with halogen atoms.

4. Process according to Claim 1, 2 or 3, characterised in that the radical R is an aliphatic radical containing from 1 to 12 carbon atoms or a substituted or unsubstituted furfuryl, benzyl or phenyl radical, and the radicals $R^3$ and $R^4$ denote a hydrogen atom or a methyl radical, or together form with the nitrogen atom an imidazolyl ring.

5. Process according to Claim 1, 2 or 3, characterised in that R is one of the groups commonly used in peptide synthesis for protecting the amino group of amino acids.

6. Process according to any one of the preceding claims, characterised in that $R^6$ and $R^7$, which may be identical or different, denote a $C_1$ to $C_{12}$ aliphatic radical or a cycloaliphatic radical which can contain 30 carbon atoms, substituted or unsubstituted with groups containing sulphur, and, not both at the same time, a hydrogen atom or a methylthio radical.

7. Process according to Claim 4, 5 or 6, characterised in that the α-halogenated derivative is α-chloroethyl ethyl carbonate, α-chloroethyl tert-butyl carbonate, α-chloroethyl furfuryl carbonate, α-chloroethyl benzyl carbonate, α-chloroethyl phenyl carbonate or α-chloroethyl 2,3-dihydro-2,2-dimethyl-7-benzofuranyl carbonate, 1,2,2,2-tetrachloroethyl tert-butyl carbonate, α-chloroethyl S-ethyl thiocarbonate, α-chloroethoxyimidazole, 1,2,2,2-tetrachloroethyl N-methylcarbamate, 1,2,2,2-tetrachloroethyl 2-trimethylsilylethyl carbonate, or 2-methyl-2-(methylthio)propanal 0-[α-chloroethyloxy-carbonyl]oxime.

8. Process according to any one of the preceding claims, characterised in that the radicals $R^1$ and $R^2$ of the amino compound, which may be identical or different, denote a hydrogen atom, an aliphatic radical containing from 1 to 20 carbon atoms, a cycloaliphatic or araliphatic radical containing up to 50 carbon atoms, or together form with the nitrogen atom a piperidino, morpholino or imidazolyl ring, the radical or ring being substituted or unsubstituted with acid, alcohol, ester, ether, mercapto or amino groups.

9. Process according to any one of the preceding claims, characterised in that $R^1$ and $R^2$ are the common radicals of natural or synthetic amino acids.

10. Process according to any one of the preceding claims, characterised in that the amine is methylamine, diethylamine, di-n-butylamine, isobutylamine, n-octylamine, ethanolamine, benzylamine, imidazole, hexamethyleneimine, morpholine, diethanolamine, N-methyl-N-benzylamine, piperidine, L-phenylalanine, L-proline, glycine, L-tyrosine, L-serine, L-aspartic acid, ethyl glycinate, phenylglycine or proline.

11. Process according to any one of the preceding claims, characterised in that the reaction is performed in the presence of one or more solvents which are inert with respect to the reagents.

12. Process according to any one of the preceding claims, characterised in that the solvents are chosen from chlorinated aliphatic solvents, cyclic or acyclic ethers, alcohols, acetone, pyridine, acetonitrile or dimethylformamide.

13. Process according to Claim 11 or 12, characterised in that the solvent medium contains water.

14. Process according to any one of the preceding claims, characterised in that the acceptor for acid is an organic or inorganic base.

15. Process according to any one of the preceding claims, characterised in that the acceptor for acid is sodium hydroxide or potassium hydroxide, sodium sulphite, sodium carbonate or bicarbonate or potassium carbonate or bicarbonate, magnesium oxide, a tertiary amine or the starting amine of formula

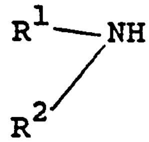

$R^1$ and $R^2$ having the significance above.

16. Process according to any one of the preceding claims, characterised in that the tertiary amine is triethylamine, pyridine or N,N-dimethylaniline.